# EUROPEAN PATENT SPECIFICATION

(11) **EP 1 541 679 B1**
(45) Date of publication and mention of the grant of the patent: **23.12.2009**
(21) Application number: 03797539.8
(22) Date of filing: 01.09.2003
(51) Int. Cl.: C12N 15/00, C12Q 1/00, C07K 19/00, C12N 5/10, G01N 33/68, G01N 33/48

(54) **METHOD OF ANALYZING ORGANELLE-LOCALIZED PROTEIN AND MATERIALS FOR ANALYSIS**
VERFAHREN ZUR ANALYSE EINES IN ORGANELLEN LOKALISIERTEN PROTEINS UND MATERIALIEN ZUR ANALYSE
PROCEDE D'ANALYSE D'UNE PROTEINE LOCALISEE DANS UN ORGANITE ET MATIERES SERVANT A CETTE ANALYSE

(30) Priority: 18.09.2002 JP 2002272043
(43) Date of publication of application: 15.06.2005
(73) Proprietor: Japan Science and Technology Agency, Kawaguchi-shi, Saitama 332-0012 (JP)
(72) Inventor: UMEZAWA, Yoshio, Shinjuku-ku, Tokyo 162-0063 (JP); OZAWA, Takeaki, Matsudo-shi, Chiba 270-2231 (JP)
(74) Representative: Owen, Deborah Jane
(86) International application number: PCT/JP2003/011156
(87) International publication number: WO 2004/027057

(56) References cited:
- EP-A- 1 141 250
- EP-A1- 1 229 330
- WO-A1-00/71701
- SIMPSON JEREMY C ET AL: "Systematic subcellular localization of novel proteins identified by large-scale cDNA sequencing" EMBO REPORTS, vol. 1, no. 3, September 2000 (2000-09), pages 287-292, XP002377400 ISSN: 1469-221X
- OZAWA T ET AL: "Detection of protein-protein interactions in vivo based on protein splicing" CURRENT OPINION IN CHEMICAL BIOLOGY 01 OCT 2001 UNITED KINGDOM, vol. 5, no. 5, 1 October 2001 (2001-10-01), pages 578-583, XP002377401 ISSN: 1367-5931
- ZIMMER MARC: "Green fluorescent protein (GFP): applications, structure, and related photophysical behavior." CHEMICAL REVIEWS. MAR 2002, vol. 102, no. 3, March 2002 (2002-03), pages 759-781, XP002377402 ISSN: 0009-2665
- OZAWA T. ET AL.: 'A fluorescent indicator for detecting protein-protein interactions in vivo based on protein splicing' ANAL. CHEM. vol. 72, 2000, pages 5151 - 5157, XP002951972
- OZAWA T. ET AL.: 'Protein splicing-based reconstruction of split green fluorescent protein for monitoring protein-protein interactions in bacteria: Improve sensitivity and reduced screening time' ANAL. CHEM. vol. 73, 2001, pages 5866 - 5874, XP002243311

### SEQUENCE LISTING

< 110> Japan Science and Technology Corporation
< 120> Analysis method for organella-localized protein and materials therefore
< 130> NP02376
<140>
   <141>
<160>2
< 170> PatentIn Ver. 2.1
<210> 1
   <211> 6
   <212> PRT
   <213> Artificial sequence
<220>
   <223> Description of artificial sequence: Synthetic oligopeptide
<400> 1
   Lys Phe Ala Glu Tyr Cys
   1 5
<210> 2
   <211> 6
   <212> PRT
   <213> Artificial sequence
<220>
   <223> Description of artificial sequence: Synthetic oligopeptide
<400> 2
   Cys Phe Asn Lys Ser His
   1 5
<210> 3
   <211> 25
   <212> DNA
   <213> Artificial sequence
<220>
   <223> Description of artificial sequence: Synthetic oligonucleotide
<400> 3
   AGGACCTTAC ACAGTCCTGC TGACC 25
<210> 4
   <211> 25
   <212> DNA
   <213> Artificial sequence
<220>
   <223> Description of artificial sequence: Synthetic oligonucleotide
<400> 4
   GCCCTCGCCG GACACGCTGA ACTTG 25
<210> 5
   <211> 28
   <212> DNA
   <213> Artificial sequence
<220>
   <223> Description of artificial sequence: Synthetic oligonucleotide
<400> 5
   CCGCCCTCAA AGTAGACGGC ATCGCAGC 28
<210> 6
   <211> 22
   <212> DNA
   <213> Artificial sequence
<220>
   <223> Description of artificial sequence: Synthetic oligonucleotide
<400> 6
   CGCCGTCCAG CTCGACCAGG AT 22

## Description

### Technical Field

The invention of the present application relates to a method for analyzing organelle-localized protein and a material for analysis. More particularly, the present invention relates to a simple and accurate method for analyzing a protein localized in various types of organelle of a eukaryotic cell and a material used in such method.

### Background Art

One of the most distinct features of eukaryotic cells, in particular mammalian cells, is that each protein is localized in each organelle. Such protein localization is closely related to the function of a protein such that localization of a certain protein is often an essential indicator for determining its function. Therefore, by analyzing intracellular localization of a protein, its function may be identified, and furthermore, a new biological significance of such protein may be formulated.

The following techniques are known as prior art for the analysis of organelle-localized protein:
(i) A method comprising cell fractionation technique and two-dimensional electrophoresis/ mass spectrometry (non-patent reference 1). In this method, cells are fractionated for each organelle, proteins expressed in each organelle are compared after two-dimensional electrophoresis and identified by mass spectrometry of the organelle specific proteins, and is useful as a method for systematic analysis of proteins. However, technique (i) relies on the yield and concentration of each intracellular organelle, and more importantly, cannot be applied to organelles for which fractionation and purification are difficult.
(ii) Expression cloning (non-patent references 2 and 3). In this method, a test protein to which a transcription factor has been linked is introduced into a cell integrated with a reporter molecule whose expression is activated in the cell nucleus, and the signal from the reporter molecule is detected. If a test protein contains a functional nuclear localization signal, the test protein and the transcription factor enter the cell nucleus and a signal of the reporter molecule can be detected. However, technique (ii) cannot be applied to organelles other than the nucleus because expression of the reporter molecule relies on the intranuclear transcription factor.
(iii) Visual screening (non-patent references 4 to 6 and 9 part VII.A "Fusion Tags"). In this method, a fusion protein of a test protein and a fluorescent protein that emits a signal is expressed in a higher eukaryotic cell and intracellular localization of the test protein is determined by observing the fluorescence signal of the fluorescent protein under a microscope. Although technique (iii) is a powerful tool for identifying various organelle-localized proteins, analysis and identification of intracellular localization of the fluorescent protein under fluorescence microscopy is time-consuming and requires excessive labor.

Meanwhile, the inventors of the present application have invented a method for analyzing interaction between two proteins (protein-protein interaction), which utilizes the principle of protein splicing, and a probe for such method (non-patent references 7, 8, 9 part VII.I "Protein-Protein Interactions", and 10), and have filed an application for patent (patent reference 1). *In vivo* production and screening of cyclic peptide libraries using an intein-based protein splicing method has also been disclosed (patent reference 2). The cyclic peptides may be targeted to particular cellular locales using, for example, signal peptides.
International publication number WO 02/08766 brochure
European publication number EP 1141250
Lopez, M. F. and Melov, S., Circ. Res. 2002, 90, 380-389
Ueki, N. et al., Nature Biotechnol. 1998, 16, 1338-1342
Rhee, Y. et al., Nature Biotechnol. 2002, 18, 433-437
Bejarano, L. A. and Gonzacz, C. J., Cell Sci. 1999, 112, 4207-4211
Misawa, K. et al., Proc. Natl. Acad. Sci. USA 2000, 92, 9146-9150
Simpson, J. C. et al., EMBO Report 2000, 3, 287-292
Gimble, F. S. Sci. Biol. 1998, 5, R251-256
Ozawa, T. et al., Anal. Chem. 2001, 73, 5866-5874
Zimmer, M., Chem. Rev. 2002, 102, 759-781
Ozawa, T. and Umezawa, Y., Current Opinion in Chemical Biology 2001, 5, 578-583

As mentioned above, the conventional techniques (i) to (iii) for analyzing organelle-localized protein are problematic in that the type of organelle that can be analyzed is limited; they require excessive labor and time for analysis, and the like. Therefore, these were inappropriate particularly for wide range screening for large-scale cDNA libraries (high-throughput screening).

The invention of the present application has been accomplished in view of the above-mentioned circumstances, and aims at providing a novel method by which protein localization can be analyzed by simple and accurate means, which is applicable to all organelles and a material for analysis to be used in this method.

### Disclosure of the invention

In order to solve the above-mentioned problems, the present application provides the following inventions (1) to (14).
(1) A method for analyzing an organelle-localized protein, which enables one to determine whether or not a test protein localizes to an organelle, comprising the following steps:
   (a) a step of introducing a fusion peptide (a), which comprises one half-peptide of an intein, one half-peptide of a fluorescent protein and an organelle-targeting signal peptide, into a eukaryotic cell;
   (b) a step of introducing a test protein bound to a fusion peptide (b), which comprises the other half-peptide of the fluorescent protein and the other half-peptide of the intein, into the eukaryotic cell; and
   (c) a step of detecting fluorescence signal emitted by the fluorescent protein.
(2) The analysis method according to the above-mentioned invention (1), wherein, in step (a), two or more types of fusion peptide (a), each comprising one half-peptide of different fluorescent proteins and different organelle-targeting signal peptides, are introduced into a eukaryotic cell; in step (b), two or more types of fusion peptides (b), each comprising the other half-peptide of the different fluorescent proteins, and each bound to a test protein, is introduced into the eukaryotic cell; and in step (c), the fluorescent signal is detected.
(3) The analysis method according to the above-mentioned invention (1) or (2), wherein, in step (a), the fusion peptide (a) is introduced into a eukaryotic cell by transfecting a recombinant vector (A) that expresses the fusion peptide (a), into the eukaryotic cell.
(4) The analysis method according to the above-mentioned invention (1) or (2), wherein, in step (b), the test protein and the fusion peptide (b) are introduced into a eukaryotic cell by transfecting a recombinant vector (B), which expresses the fusion peptide (b) and the test protein as a unit, into the eukaryotic cell.
(5) A fusion peptide (a), which comprises a half-peptide of an intein, a half-peptide of a fluorescent protein and an organelle targeting signal peptide.
(6) A fusion peptide (b), which comprises a half-peptide of a fluorescent protein and a half-peptide of an intein.
(7) A recombinant vector (A), which expresses a fusion peptide (a) comprising a half-peptide of an intein, a half-peptide of a fluorescent protein and an organelle targeting signal peptide.
(8) A recombinant vector (B), which expresses a fusion peptide (b) comprising a half-peptide of a fluorescent protein and a half-peptide of an intein, and an arbitrary test protein bound thereto.
(9) A probe set for analyzing organelle-localized protein, comprising the fusion peptide (a) of the above-mentioned invention (5) or the recombinant vector (A) of the above-mentioned invention (7), and the fusion peptide (b) of the above-mentioned invention (6) or the recombinant vector (B) of the above-mentioned invention (8).
(10) The probe set according to the above-mentioned invention (9), wherein the fusion peptide (a) or the fusion peptide (a) expressed by the recombinant vector (A) comprises two or more types of fusion peptides, each fusion peptide comprising one half-peptide of a fluorescent protein having different signal characteristics and a different organelle targeting signal peptide; and the fusion peptide (b) comprises two or more types of fusion peptides, each fusion peptide comprising the other half of the fluorescent protein.
(11) A eukaryotic cell, containing a fusion peptide (a), which comprises a half-peptide of an intein, a half-peptide of a fluorescent protein and an organelle targeting signal peptide.
(12) A cell kit, comprising two or more of the eukaryotic cells of the above-mentioned invention (11).
(13) A eukaryotic cell, comprising two or more types of fusion peptide (a), wherein each fusion peptide comprises one half-peptide of a fluorescent protein and an organelle targeting signal peptide, the fluorescent protein of each fusion peptide have different signal characteristics and the organelle targeting signal peptide of each fusion peptide target different organelle.
(14) A cell kit, comprising two or more of the eukaryotic cells of the above-mentioned invention (13).

In other words, the analysis methods according to the above-mentioned inventions (1) to (4) are based on the reconstruction of a fluorescent protein by protein splicing of an intein (non-patent references 7 and 8), and can be implemented by using the various materials according to the above-mentioned inventions (5) to (14).

Incidentally, in the invention of the present application, the terms "protein" and "peptide" are used to indicate those that are isolated and purified from a cell, those produced by genetic engineering, those synthesized, or their biologically active equivalent, namely amino acid polymers formed by a series of amide linkage known as peptide bond.

A "test protein" is a protein expressed in an organism cell (especially a eukaryotic cell) whose function is known or unknown and, especially, a protein whose organelle localization is unknown. A test protein whose amino acid sequence is known is preferable and a test protein whose base sequence encoding the amino acid sequence is known is more preferable. This test protein may be, for example, selected from a known protein library and used, or may be a protein produced by genetic engineering from each cDNA clone of a cDNA library (an existing library or a cDNA library prepared from a total RNA of an arbitrary cell) and used.

A "eukaryotic cell" is a yeast cell, an insect cell, an animal cell or the like, and especially, a cell of a mammal including human.

An "organelle" exists inside a eukaryotic cell membrane and is a structural unit which shares various functions of the cell. This includes, for example, cell nucleus, mitochondrion, endoplasmic reticulum, Golgi body, secretory granule, secretory vesicle, lysosome, phagosome, endosome, peroxisome and the like.

An "organelle targeting signal peptide" may be a full-length protein specifically localized in each organelle, or a transition signal (or localization signal) peptide that exists in such localized protein and functions for the localization of the protein; known proteins or peptides may be used. For example, as a nuclear targeting signal peptide, an intranuclear protein (for example, histone, viral protein and the like) or its partial signal peptide may appropriately be used. For organelle such as mitochondrion, endoplasmic reticulum, Golgi body and peroxisome, an enzyme which is used as a marker enzyme for each organelle in methods such as cell fractionation (for example, cytochrome c oxidase for mitochondrion, glucose-6-phosphatase for endoplasmic reticulum, galactosyltransferase for Golgi body, catalase for peroxisome and the like) or a signal peptide thereof can be used. Amino acid sequence and base sequence of the polynucleotide encoding such amino acid for such an organelle targeting peptides, may be obtained from known protein databases (for example, URL: HYPERLINK 'http://www.ncbi.nlm. nih.gov/Entrez' http://www.ncbi.nlm.nih.gov/Entrez).

An "intein" is an internal protein segment which is excised by splicing from a protein after translation, and may be a wild-type intein derived from various types of organisms or the "functional domain" that is involved in protein splicing. Specific examples of an intein include, but are not limited to, VMA derived from Saccharomyces cerevisiae, Candida tropiallis, Thermoplasma asidophilum or the like, RecA or pps1 derived from Mycobacterium tuberculosis, DnaB or DnaE derived from Synechocystis, and the like. The types of inteins that are applicable, as well as their amino acid sequences and base sequences may be found in InBase: the Intein Database (Nucleic Acids Res. 2002, 30(1), 383-384; URL: HYPERLINK 'http://www.neb.com/neb/inteins.html' http://www.neb.com/neb/ inteins.html).

A "fluorescent protein" is a protein which emits fluorescence when it is irradiated with an excitation light, or its functional domain. Examples of the fluorescent protein include green fluorescent protein (GFP) derived from aequorea victoria, its mutants including EGFP, EYFP (yellow fluorescence), ECFP (cyan fluorescence), DsRed1 and DsRed2 (red fluorescence), green fluorescent protein hrGFP derived from Renilla and the like. Information such as the amino acid sequences of the fluorescent proteins and the base sequences encoding them may also be obtained from known protein databases (for example, URL: HYPERLINK http://www.ncbi.nlm. nih.gov/Entrez' http://www.ncbi.nlm.nih.gov/Entrez).

A "half peptide" is a peptide having the C-terminal or the N-terminal amino acid sequence of each of the above-mentioned intein and fluorescence protein. When the C-terminal half-peptide and the N-terminal half-peptide are combined, a full-length protein or a functional domain of the full-length protein of the intein or the fluorescent protein is formed. When one of the half-peptides is the C-terminal side, the other half-peptide is the N-terminal side, and when one is the N-terminal side, the other is the C-terminal side. In addition, "half" does not necessarily mean half in a strict sense but rather implies that the functional domain of a protein is separated into two parts by breaking a particular amide bond.

A "fusion peptide" is a peptide in which each of the above-mentioned half-peptides or targeting signal peptide is tandemly fused and the C-terminus and the N-terminus of each peptide are connected by a peptide bond. In addition, each peptide may be connected via a "linker peptide". For example, in the above-mentioned intein VDE, a mutant in which the endonuclease domain is replaced by a flexible dodecapeptide linker is know to show high splicing activity (Cooper, A. A., Chen, Y. J., Lindorfer, M.A., and Stevens, T. H., EMBO J., 12, 2575-2583, 1993; Chong, S. and Xu, M.-Q., J. Biol. Chem., 272, 15587-15590, 1997).

Other terms and concepts used in this invention will be described in the description of embodiments and the Examples of the invention. Unless specified by reference, the various genetic engineering techniques utilized to implement the present invention may easily and reliably be conducted by those skilled in the art by referring to known publications (for example, Sambrook and Maniatis, in Molecular Cloning-A Laboratory Manual, Cold Spring Harbor Laboratory Press, New York, 1989),.

Hereinafter, embodiments of the above-mentioned inventions will be described in detail.

### Brief Description of the Drawings

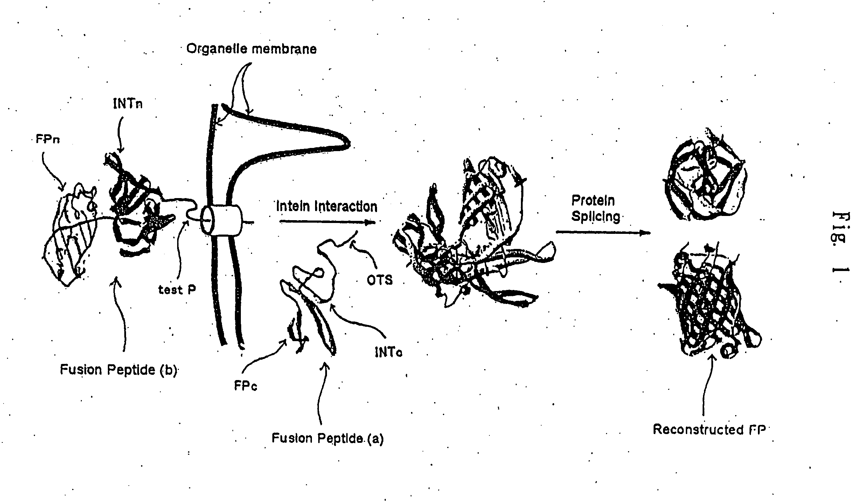

Fig. 1 is a schematic diagram showing the basic principle of the method for the present invention.

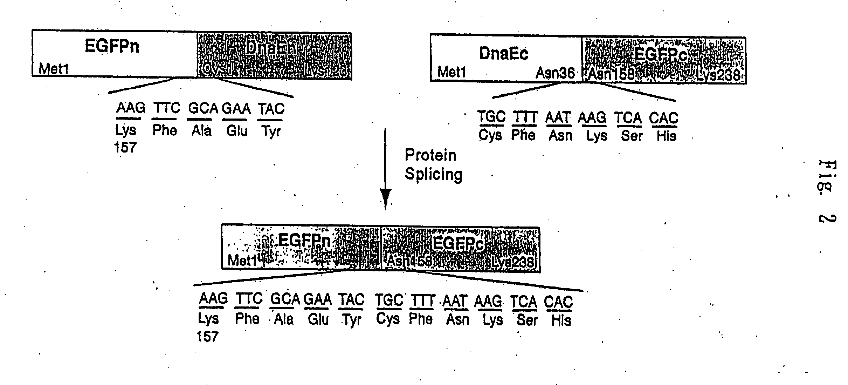

Fig. 2 is a schematic diagram showing the structures of fusion peptides (b) and (a) produced in the Examples, and the structure of EGFP reconstructed after protein splicing. The base sequences and amino acid sequences indicate linker peptide sequences between DnaEn and EGFPn, DnaEc and EGFPc, and EGFPn and EGFPc.

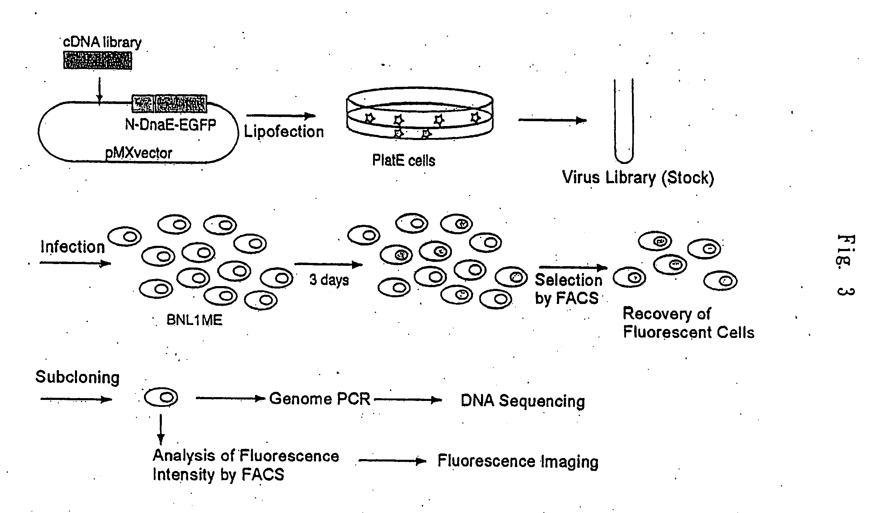

Fig. 3 is a schematic diagram of the analysis process used in the Examples.

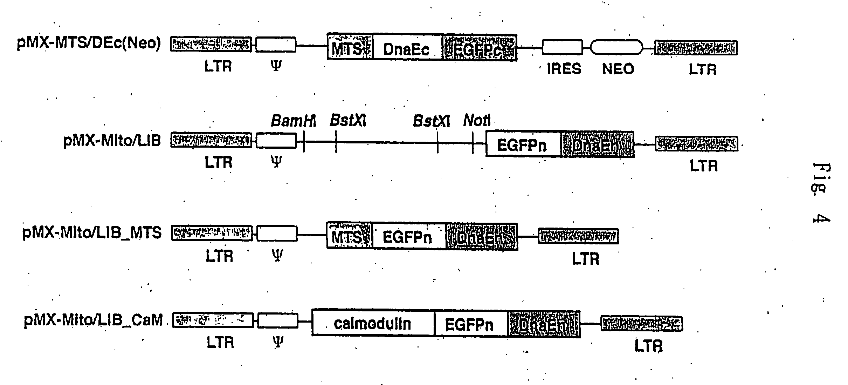

Fig. 4 is a schematic diagram showing the structures of the recombinant vectors produced in the Examples. LTR indicates long terminal repeat, ψ indicates retrovirus-packaging signal, IRES indicates internal ribosome entry site, and NEO indicates neomycin resistant gene.

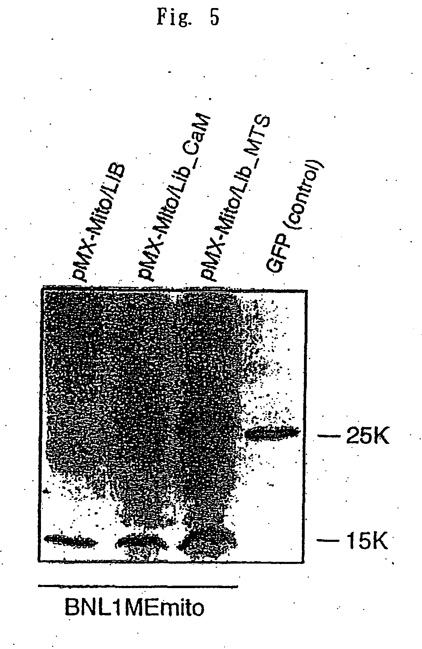

Fig. 5 shows the results of the Western Blotting analysis of whole cell lysates from BNL1MEmito cells expressing EGFPn-DnaEn tagged with calmodulin (CaM) or MTS. The analysis was performed by using a monoclonal antibody specific to EGFPc.

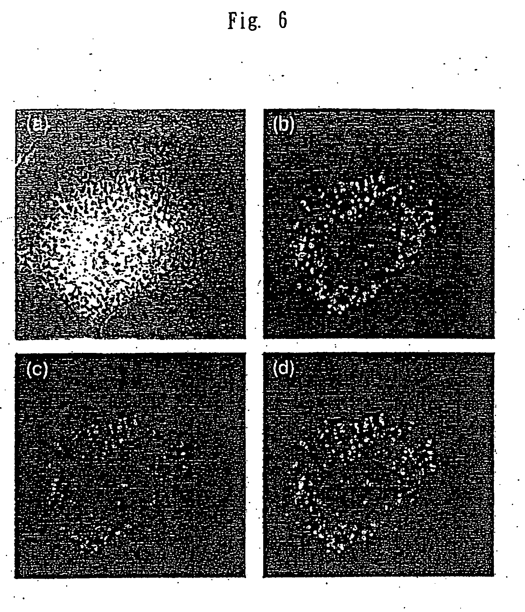

Fig. 6 shows micrographs indicating expression and localization of MTS-EGFPn-DnaEn fusion peptides in mitochondria. BNL1MEmito cells infected with pMX-Mito/LIB-MTS at an MOI value of 5 were cultured for 2 days and cells were spread on a glass-base dish. Images of live cells were taken (a; transmission) and fluorescence of EGFP was recorded by a confocal microscope (b). After imaging, mitochondria in the live cells were stained with tetramethylrhodamine ethyl ester (c). (d) is a superimposed image indicating specific localization of EGFP in the mitochondria.

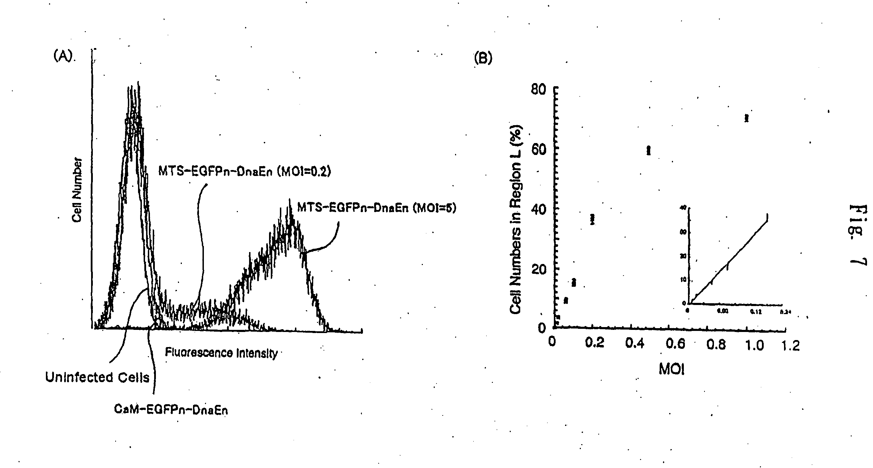

Fig. 7 shows FACS profiles of BNL1MEmito cells harboring reconstructed EGFP. In the left graph (A), BNL1MEmito cells were infected with retroviruses expressing CaM-EGFPn-DnaEn at an MOI value of 5 and MTS-EGFPn-DnaEn at an MOI value of 5 or 0.2, respectively. Uninfected cells were used as a control. The right graph (B) shows the results of measuring the retrovirus infection as the control. The single-hit kinetics of retrovirus infection is illustrated by the linear correlation of MOI versus the percentage of EGFP-positive cells in region L. All data points were obtained from 10,000 measured cells, and the measurements were repeated three times. The inset is an enlargement of the linear correlation range.

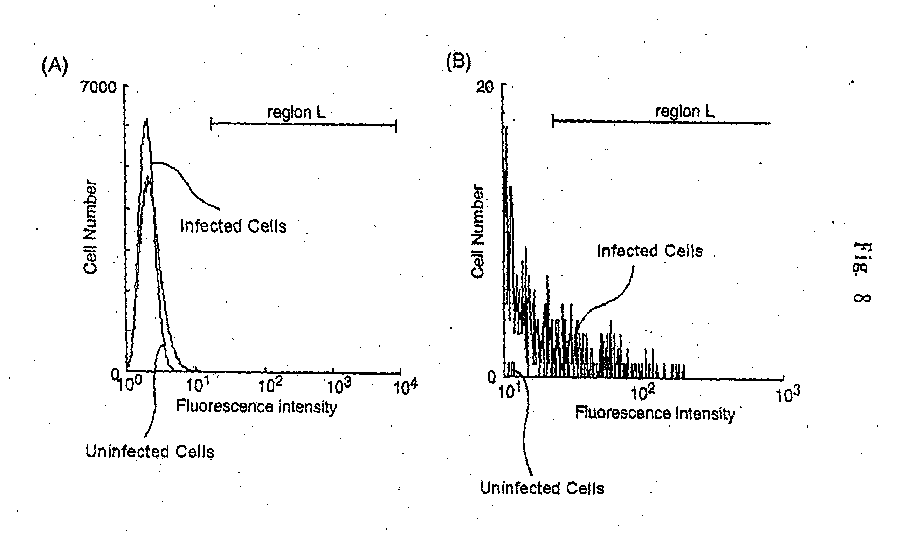

Fig. 8 shows the results of sorting fluorescent cells by FACS. (A) shows the results of sorting by FACS after BNL1ME cells were infected with the cDNA retrovirus library at an infection efficiency of 20%, incubated for 5 days and stripped off. Uninfected cells were inserted to show the background fluorescence. (B) shows enlarged FACS profiles of (A) around region L.

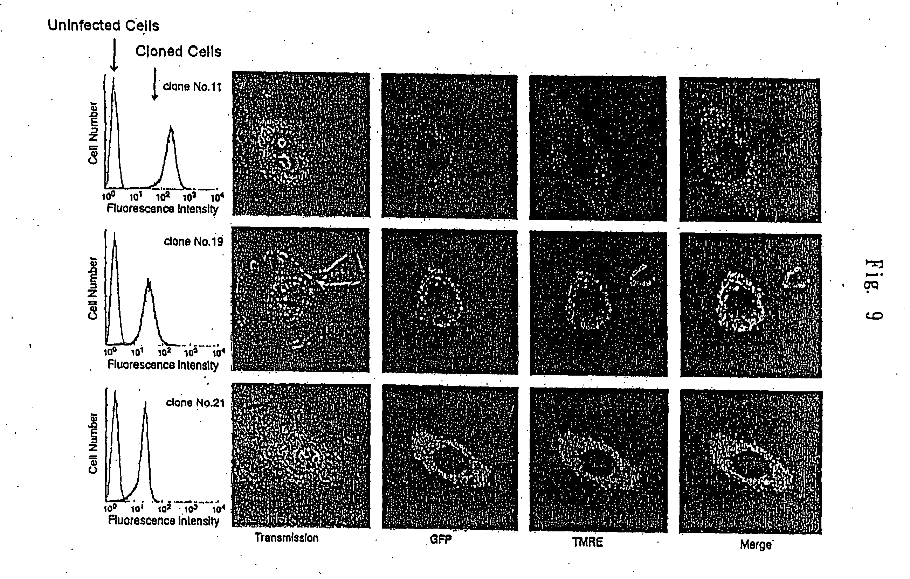

Fig. 9 shows flow cytometry profiles and fluorescent images of representative cloned cells. The graphs on the left show the results of measuring fluorescence intensities of the cloned cells and the uninfected BNL1MEmito cells by flow cytometry. Total cell counts to be analyzed were 10⁵ cells. The fluorescent images on the right show the results of confocal imaging of the live cells harboring the reconstructed EGFP after culturing each cloned cell on a glass slide. The cells were stained with TMRE to show the mitochondrial localization of individual cells. Stacked confocal images show that reconstruction of EGFP occurred in the mitochondria.

### Best Mode for Carrying Out the Invention

Invention (1) is a method for analyzing whether or not a test protein is localized in an arbitrary organelle, comprising the following steps.

Step (a): Introducing a fusion peptide (a) comprising one of the half-peptides of an intein, one of the half-peptides of a fluorescent protein and an organelle targeting signal peptide, into a eukaryotic cell.

Step (b): Introducing a test protein bound to a fusion peptide (b), which comprises the other half-peptide of the above-mentioned fluorescent protein and the other half-peptide of the above-mentioned intein, into the eukaryotic cell.

Step (c): Detecting the fluorescence signal emitted by the above-mentioned fluorescent protein.

The method of the present invention (1) can be implemented by using fusion peptide (a) (Invention (5)) and fusion peptide (b) (Invention (6)) provided by the present invention. Half-peptides of an intein and half-peptides of a fluorescent protein used for each of the fusion peptides are produced from the same intein and fluorescent protein, respectively. Further, the C-terminal half-peptides of each protein are bound together and the N-terminal half-peptides of each protein are bound together. Thus, if the fusion peptide (a) is a combination of C-terminal half-peptides, the fusion peptide (b) should be a combination of N-terminal half-peptides, or vice versa. However, for the N-terminal half-peptide and the C-terminal half-peptide of the intein to ligate in the organelle and show splicing activity, the order of combination should be the N-terminal half-peptide of the fluorescent protein (FPn) and the N-terminal half peptide of the intein (INTn) (N-FPn/INTn-C), or the C-terminal half peptide of the intein (INTc) and the C-terminal half peptide of the fluorescent protein (FPc) (N-INTc/FPc-C). Hereinafter, the invention shall be described using, as an example, the case where fusion peptide (a) is N-INTc/FPc-C and fusion peptide (b) is N-FPn/INTn-C.

The organelle targeting signal peptide (OTS) in fusion peptide (a) may be bound to the C-terminal side or the N-terminal side of N-INTc/FPc-C (N-OTS/INTc/FPc-C or N-INTc/FPc/OTS-C). In addition, the test protein (testP) may be bound to either side of fusion peptide (b) (N-testP/FPn/INTn-C or N-FPn/INTn/testP-C).

Fusion peptide (a) and fusion peptide (b)/testP can be produced by peptide-bonding of the peptide/protein through known methods. In addition, they can be produced by chemical synthesis through known solid phase synthesis methods or the like. Alternatively, they can also be produced by expressing a fusion polynucleotide prepared by connecting polynucleotides encoding each of the peptides in an in vitro transcription-translation system or an appropriate host-vector system.

For example, when the fusion peptide is produced by in vitro transcription-translation, the above-mentioned fusion polynucleotide is inserted into a vector containing RNA polymerase promoter to create an expression vector. Then, this vector is added to an in vitro translation system such as rabbit reticulocyte lysate or wheat germ extract that contains RNA polymerase corresponding to the promoter. Examples of RNA polymerase promoter include T7, T3, SP6 and the like. Examples of the vector containing such an RNA polymerase promoter include pKA1, pCDM8, pT3/T7 18, pT7/3 19, pBluescript II and the like.

When the fusion peptide is expressed in bacteria such as E. coli, an expression vector obtained by the recombination of the above-described DNA fragment to an expression vector that contains a replicable origin, promoter, ribosome binding site, DNA cloning site, terminator, and the like, is produced and the fusion peptide is isolated from the culture. Examples of the expression vector for E. coli include the pUC system, the pBluescript II, the pET expression system, the pGEX expression system and the like.

Further, when the fusion peptide is expressed in a eukaryotic cell, a recombinant vector is produced by inserting the above-mentioned fusion polynucleotide into an expression vector for eukaryotic cell having a promoter, a splicing site, a poly(A) addition site and the like and introduced into a eukaryotic cell. Thus, the fusion peptide can be expressed in a transformed eukaryotic cell. Examples of the expression vector include pKA1, pCDM8, pSVK3, pMSG, pSVL, pBK-CMV, pBK-RSV, EBV vector, pRS, pcDNA3, pMSG, pYES2 and the like. As the eukaryotic cell, mammalian cultured cells such as monkey kidney cell COS7 or Chinese hamster ovary cell CHO, budding yeast, fission yeast, a silkworm cell, a Xenopus egg cell or the like is generally used; however, any eukaryotic cell may be used as long as it can express the desired fusion peptide. To introduce an expression vector into a eukaryotic cell, a known method such as the electroporation method, the calcium phosphate method, the liposome method, and the DEAE-dextran method can be used.

After expressing the fusion peptide in a prokaryotic cell or a eukaryotic cell, the target peptide may be isolated from the culture and purified by combining known separation operations. For example, treatment with a denaturing agent such as urea or a surfactant, supersonic treatment, enzyme digestion, salt precipitation or solvent precipitation method, dialysis, centrifugation, ultrafiltration, gel filtration, SDS-PAGE, isoelectric focusing, ion-exchange chromatography, hydrophobic chromatography, affinity chromatography, reverse phase chromatography and the like may be applied.

In steps (a) and (b), to introduce the fusion peptide (a) and the fusion peptide (b)/testP into a cell, for example, an intracellular introduction method that uses lipid (BioPORTER (Gene Therapy Systems, Inc., USA), Chariot (Active Motif, Inc., USA) and the like) can be adopted. In addition, the fusion peptide can be introduced into a cell by ligating PTD (protein transduction domain) of HIV-1 TAT or PTD of Drosophila homeobox protein Antennapedia, which is a cell membrane permeable peptide, to the above-mentioned fusion peptide.

Or the target fusion peptide can be introduced into a cell by the methods (Inventions (3) and (4)) using recombinant vector (A) (Invention (7)) and recombinant vector (B) (Invention (8)) of the present invention. The methods of inventions (7) and (8) are preferable in that the introduction of the fusion peptide into a cell can be achieved more simply and surely. The recombinant vectors (A) and (B) can be produced by using an expression vector for a eukaryotic cell and a fusion polynucleotide, for which the genetic engineering production of a fusion peptide was described above. If these recombinant vectors are introduced into a eukaryotic cell by the above-mentioned known methods, the fusion peptide encoded by the fusion polynucleotide can be expressed in the cell.

In step (a), fusion peptide (a) introduced into a cell according to the above-mentioned method is transferred into a designated organelle by its OTS (Fig. 1). Furthermore, regarding fusion peptide (b)/testP, which is introduced into the cell in step (b), if testP has the designated organelle localization, it is transferred into the organelle, and interacts with fusion peptide (a) that is localized therein; then, INTn and INTc assemble, is excised by protein splicing, and FPn and FPc are reconstructed, thus emitting fluorescence signal (Fig. 1).

Therefore, by detecting the fluorescence signal of the cell in step (c), whether or not testP shows designated organelle localization may be determined. The fluorescence signal may also be detected by observing the cell through fluorescent microscopy. Alternatively, cells that emit fluorescence signal may be sorted by a fluorescence-activated cell sorting (FACS) method. This method using FACS is, due to its simplicity, a preferable method, because it enables a wide range screening (a high-throughput screening) aiming at, for example, large-scale protein libraries or cDNA libraries.

Invention (2) of the present application is an embodiment of the analysis method of the above-mentioned invention (1). In other words, in the method of invention (2), in step (a), respective fusion peptides (a) are introduced into two or more different organelles in a cell. Each fusion peptide (a) contain an organelle targeting signal peptide that targets different organelles, and the fluorescent proteins each show distinct characteristic (such as color). For example, fusion peptides (a) with half-peptides of green fluorescent protein (EGFP), yellow fluorescent protein (EYFP) and cyan fluorescent protein (ECFP) are localized in mitochondria, endoplasmic reticulum and Golgi body, respectively. Then, in step (b), fusion peptide (b) having the other half-peptide of the above-mentioned respective fluorescent proteins, and the test protein bound thereto are introduced into the cell. Thus, by detecting the absorbance corresponding to each color (green, yellow or cyan) or color change of the fluorescence signal emitted by the cell, the location at which the test protein localizes, i.e. mitochondria, endoplasmic reticulum or Golgi body, can be determined.

Incidentally, inventions (1) and (2) may be performed efficiently by using the probe sets provided by the present application (inventions (9) and (10)). Furthermore, by using the cells provided by the present application (inventions (11) and (13)), step (a) can be omitted. In addition, these cells may be made into cell kits (Inventions (12) and (14)) comprising two or more of these cell populations. The cell kit of invention (12) may consist of a plurality of cell populations wherein all of the cells contain fusion peptide (a) in the same organelle, or may consist of a plurality of cell populations wherein each cell contains fusion peptide (a) in varying organelles. The cell kit of invention (14) may consist of a plurality of cell populations wherein all cells contain fusion peptide (a) in the same two or more organelles, or may consist of a plurality of cell populations wherein each cell contains fusion peptide (a) in varying two or more organelles. Also, when the cell is a floating cell, each cell may be suspended in an appropriate liquid medium, and when a cell is an adhesive cell, the cell may be immobilized in the form of a "cell chip". Furthermore, cells that constitute such cell kits may be the same kind of cells, or may be different types of cells. For example, a cell kit may be composed of a combination of normal cells and disease cells (for example, cancer cells or the like).

Hereinafter, the invention of the present application will be described in further detail with reference to the following Examples; however, the present invention is not limited to the following Examples.

### Examples

### 1. Methods

### 1.1 Production of Expression Vector

The enhanced EGFP cDNA of its amino acid 1-157 was amplified by polymerase chain reaction (PCR) to introduce Lys-Phe-Ala-Glu-Tyr-Cys (SEQ ID NO: 1) to the C-terminus of spEGFP. This cDNA was fused to the cDNA of the N-terminal splicing domain of the DnaE intein and subcloned in the prokaryotic vector Bluescript. The PCR product was sequenced to confirm the base sequence and was subcloned into pMX vector at SalI restriction sites. To create fusion peptide (b) composed of the N-terminal half-peptide of EGFP (EGFPn) and the N-terminal half-peptide of DnaE (DnaEn) bound with a mitochondrial targeting signal peptide (MTS) or calmodulin, the cDNA was amplified by PCR to introduce BamHI (5') and NotI (3') restriction sites. The PCR products were inserted into pMX-Mito/LIB in frame and their sequences were verified (see Fig. 2).

### 1.2 Selection of Stable Clone

The cDNA of the C-terminal half-peptide of DnaE (DnaEc) bound with MTS was amplified by PCR. The cDNA of the carboxyl-terminal half of EGFP corresponding to 158-238 was amplified by PCR with extending the peptide of Cys-Phe-Asn-Lys-Ser-His (SEQ ID NO: 2) to the amino terminus. These two PCR products were ligated at MunI sites to form fusion peptide (a) and subcloned in the pBluescript (see Fig. 2). The product was sequenced to confirm the base sequence and subcloned into pMX vector at BamHI (5') and SalI (3) restriction sites. After amplification in DH5α' Escherichia coli, the fusion gene was transfected into PlatE cells with Lipofectamine Plus (Invitrogen). After two days of culture, high-titer retroviruses were collected and transfected into BNL1ME cells. Stable expressing cells were obtained after approximately 10 days of selective culture in G418 (Invitrogen) containing the growth medium (see Fig. 3).

### 1.3 Construction of cDNA Library

Poly(A)+ RNA was purified from 1 x 10⁸ BNL1ME cells using a FastTrack kit (Invitrogen). cDNA was synthesized from the Poly(A)+ RNA by random hexamers using a cDNA synthesis kit (Invitrogen). The resulting cDNAs were size-fractionated through column chromatography and agarose gel electrophoresis, and cDNA fragments of 600 kbp or longer were extracted from the agarose gel by using a Qiaex II kit (Qiagen). The cDNA fragments were inserted into BstXI sites of pMX-Mito/LiB by using BstXI adapters (Invitrogen). Next, the ligated DNA was ethanol-precipitated and then transfected into DH10B-competent cells (Invitrogen). Plasmid DNA was purified by using Qiaex (Qiagen) after 200 mL of culture for 16 hours. The plasmids were transfected into packaging cell line PlatE with Lipofectamine Plus (Invitrogen). After two days of culture, high-titer retroviruses were collected (see Fig. 3).

### 1.4 Sorting Strategy

Subconfluent (70%) BNL1ME cell layers were infected with the constructed retrovirus library with an infection efficiency of 20% or less. The infection efficiency was estimated by a control experiment using pMX-EGFP. The cells were detached 48 hours after infection and spread into four 6-cm-diameter dishes. After a 72-hour incubation, the cells were stripped with tripsin-EDTA and dissolved in a PBS buffer (Gibco BRL). FACS analysis was performed on an ALTRA flow cytometer (Beckmann Coulter) for sorting GFP-positive single cells. These cells were incubated in a 96-well plate or spread into a 10-cm-diameter dish followed by subcloning using chips (see Fig. 3).

### 1.5 Identification of Integrated cDNA

Genomic cDNAs extracted from BNL1ME clones were amplified by the nested PCR method to recover the integrated cDNAs. As the primers, a set of 5'-AGGACCTTACACAGTCCTGCTGACC-3' (SEQ ID NO: 3) and 5'-GCCCTCGCCGGACACGCTGAACTTG-3' (SEQ ID NO: 4), and a set of 5'-CCGCCCTCAAAGTAGACGGCATCGCAGC-3' (SEQ ID NO: 5) and 5'-CGCCGTCCAGCTCGACCAGGAT-3' (SEQ ID NO: 6) were used. The PCR was run for 30 cycles (30 sec. at 98°C for denaturation, 30 sec. at 58°C for annealing and 2 min. at 72°C for extension) using LA Taq polymerase (Takara Shuzo). The resulting second PCR fragments were sequenced using a BigDyeTerminator Cycle Sequencing Kit (Applied Biosystems) and were analyzed by an automatic sequencer (310 Genetic Analyzer; Applied Biosystems) (see Fig. 3).

### 1.6 Gene Sequence and Functional Analysis of Genes

Each cDNA sequence was compared with the cDNA sequences in databases including GenBank, PDB, SwissProt, PIR, PRF using BLASTn. Orientation of the cDNA strands was identified by the RIKEN clone sets, which were categorized in several stages, and their functions were analyzed. Homology analysis was performed using the Blast program.

### 1.7 Imaging Fluorescence Signal

BNL1ME clones were spread on a glass-base dish and incubated for 24 hours in the presence of the growth medium. The medium was replaced by a PBS solution supplemented by 5% FCS and the live cells were directly imaged using a confocal laser-scanning microscope (Carl Zeiss). After imaging, mitochondria were stained with tetramethylrhodamine ethyl ester (TMRE; Molecular Probes). The final concentration of the TMRE in the PBS buffer was adjusted at 1 µM. Incubation was performed for 10 minutes. The cells were irradiated with a wavelength of 543 nm and the image was taken through a 560 nm LP filter.

### 2. Results

### 2.1 Selective and Highly Sensitive Detection of Mitochondrial Proteins

For performing this library screening accurately, the following two requirements need to be fulfilled. 1) The fluorescence intensity of EGFP reconstituted in mitochondria is highly sensitive and strong enough to be detected by FACS analysis. 2) The cells that include a protein in the presence of MTS can be selectively separated and collected from those in the absence of MTS. To examine this selective and highly sensitive detection, proteins for which the intracellular localization are well characterized were tested in mouse liver cells (BNL1ME). The plasmid pMX-MTS/DEc(Neo), which encodes cDNAs corresponding to the C-terminal half-peptides of EGFP and DnaEc, and a mitochondrial targeting signal corresponding to the precursor of subunit VIII of cytochrome C oxidase, was constructed (Fig. 4). At the splicing junction, cDNA sequences encoding additional 5 amino acids were inserted for efficient splicing to occur (Fig. 2) (Evans, J. et al., J. Biol. Chem. 2000, 275, 9091-9094). The plasmid was converted into retroviruses and they were infected into BNL1ME cells. A stable cell line expressing the corresponding test protein in mitochondria was developed (BNL1MEmito). As the test protein, a known cytosolic protein, calmodulin, or a signal peptide, MTS, was used. Their cDNAs were bound to cDNAs encoding EGFPn and DnaEn, and their fusion peptides were expressed in the BNL1MEmito cells (Fig. 4). Western blots of the cells revealed that protein splicing occurred to produce native EGFP whose molecular weight is slightly larger than that of wild-type EGFP, reflecting the addition of the 10 amino acids at the splicing junction (Fig. 5). To confirm that the protein splicing occurred in mitochondria, fluorescent images of live BNL1MEmito cells were examined. The localization of EGFP was found substantially the same as the case of the mitochondria stained with a cell-permeable mitochondrion-selective dye, tetramethylrhodamine ethyl ester (Fig. 6). In addition, it was confirmed that EGFP formation following protein splicing specifically occurred for the fusion peptide tagged with MTS at the N terminus.

To ensure that the fluorescence intensity of the reconstructed EGFP is strong enough to isolate fluorescent cells by a cell sorter, BNL1MEmito cells were infected at various multiplicities of infection (MOI, which is defined as the number of cDNAs per cell) with retroviruses producing MTS-EGFPn-DnaEn. Control of the MOI is particularly important because multiple integration of cDNAs in the BNL1MEmito cells may result in the isolation of false positive cDNAs after cell sorting. Therefore, it was needed to control the infection efficiency as a single-hit event. To assess this, 48 hours after the infection with various MOIs, the number of the cells including the reconstructed EGFP was evaluated by flow cytometry. At MOI of 5, all of the cells showed strong fluorescence (Fig. 7). At MOI of 0.01, 1.6 ± 0.1%; at MOI of 0.02, 3.6 ± 0.3%; at MOI of 0.06, 9.4 ± 0.6%; at MOI of 0.1, 15.4 ± 1.1%; at MOI of 0.2, 36.7 ± 1.4%; at MOI of 0.5, 60.5 ± 1.3%; and at MOI of 1.0, 71.1 ± 1.0% of the cells showed fluorescence. The fluorescent cells increased linearly with increasing MOI in the MOI range of 0 to 0.2, demonstrating that, in the MOI range of 0 to 0.2, infection occurred as one cDNA per cell. At this single-hit infection, the magnitude of fluorescence intensity of EGFP became sufficient enough to separate the cells between the presence and absence of MTS, as evidenced by the breadth of the two peaks of fluorescence intensity. These data show that the amounts of reconstructed EGFP in a single BNL1MEmito cell was sufficient to allow highly sensitive detection of mitochondrial proteins and its selective isolation using a cell sorter.

### 2.2 Selection of Mitochondrial proteins from cDNA Libraries

The selective isolation of genes encoding mitochondrial protein from large cDNA libraries was investigated. The cDNAs derived from BNL1MEmito cells were cloned into two BstXI sites upstream of cDNA fragments of EGFPn and DnaEn, thereby creating cDNA-EGFPn-DnaEn fusion libraries (Fig. 3). The order of the tandem fusion fragments, cDNA-EGFPn-DnaEn, was crucial for analyzing its intracellular localization, because most MTSs are known to attach to the amino-terminal end of a mitochondrial protein (Roise, D. et al., EMBO J. 1988, 7, 649-653; Von Heijne, G. EMBO J. 1986, 5, 1335-13429, 10). The cDNA library thus constructed contained 1.1 x 10⁶ independent clones, with the size of cDNAs averaging 1.4 kbp. The library was converted to retroviruses by using a high-titer retrovirus packaging cell line, Plat-E cells (Morita, S. et al., Gene Therapy 2000, 7, 1063-1066).

As a pilot experiment, 1 x 10⁷ cells were infected with 50 µL, of the retroviral supernatant to achieve 20 % infection efficiency. The fluorescence intensity of the 1 x 10⁵ cells was measured by FACS analysis 3 days after the infection. The population of the infected cells consisted of a mixture of cells in the presence and absence of the reconstructed EGFP (Fig. 8). The percentage of the fluorescent cells in region L was found to be 0.089 ± 0.008% (n=10) of the total cells.

Next, a population of fluorescent cells in region L was collected by FACS analysis. Data rate, defined as the number of cells analyzed per second, was controlled to be (1.0 ± 0.1) x 10³. Upon setting this data rate, 10⁷ cells could be examined within a few hours. In this experiment, a total of 1 x 10³ cells were counted as a fluorescent cell in region L, but half of the cells were aborted. The fluorescent cells that were actually collected were therefore 500 to 1000 cells, indicating that this EGFP reconstruction technology in combination with FACS enables high-speed collection of MTS-tagged fusion peptides.

Further, to assess the accuracy of the cell sorting, the fluorescence intensity and intracellular localization of each isolated clone were analyzed. If the cDNA was integrated in the host genome, the corresponding protein should be constitutively expressed in the BNL1MEmito cell and therefore EGFP reconstruction should be kept in the mitochondria. In order to confirm this, fluorescence intensities of the collected 200 clones were analyzed by FACS, among which 169 clones showed fluorescence of various intensities (Fig. 9). The rest of the 31 clones of which cDNA could not be recovered by genomic PCR did not fluoresce, indicating that the cDNA was not integrated in the nuclear genome or that the cDNA, after being integrated, dropped out of the nuclear genome during cell division. Next, 100 clones of the fluorescent cells were randomly selected and the intracellular localization of reconstructed EGFP was examined. The EGFP was found to be localized exclusively in mitochondria (Fig. 9), demonstrating that in these cells, a cDNA encoding a mitochondrial protein was integrated in each clone and the cDNA sequence was readily detectable.

### 2.3 Analysis of Individual cDNA Clones

To characterize the individual cDNA, the nuclear genome was extracted from each clone and the integrated cDNA was recovered by PCR amplification, which was subjected to sequence analysis. Of the first 150 clones analyzed, the expressed sequence tags (ESTs) obtained included 32 tags that occurred once and 28 tags that were identified multiple times. In a total of 60 non-redundant cDNAs, 56 clones were identified in GenBank. The other 4 genes were identified newly, and were found to include mitochondrial targeting signals. The localization of each novel gene product in the mitochondria was confirmed by confocal microscopy.

Of the total 56 clones existing in GenBank, a number of well-characterized mitochondrial proteins were identified, which included, for example, Acad1, Gcdh, Cox5b, ATP synthase, Ucp2, maleate dehydrogenase (Table 1). All of these proteins existed in the mitochondrial matrix or inner membrane. Of the clones for which characteristics were unknown, functions of some gene products were newly annotated as follows:

For example, cDNA derived from clone No. 10 was identified in public sequence database DDBJ (RIKEN full-length cDNA clones) (Hayashizaki, Y. et al., Nature 2001, 409, 685-690). Reading frames and expected start codons of cDNAs obtained from clone No. 10 completely matched those found in the database. Homology analysis using public databases showed that there was a 23% homology at the DNA level between the cloned cDNA fragment and putative cytochrome c oxidase assembly protein derived from Schizosaccharomyces pombe. Therefore mouse clone No. 10 belongs to a cytochrome c oxidase assembly protein or a protein that shows related functions. Similarly, the cDNA derived from clone No. 92 was found to be a 76% homologue of the cDNA of human mitochondrial 28S ribosomal protein (S18-1). This high homology and its mitochondrial localization obtained in this experiment confirmed that the cDNA for clone No. 92 is a mouse mitochondrial ribosomal protein. Another ribosomal protein S18 (clone No. 51) has already been identified as a mouse ribosomal protein, but its localization had not been discussed in detail.

MTS is composed of some 20 to 60 amino acid residues that have the potential to form amphiphilic α-helices with one hydrophobic face and one positively charged face. The fact that basic and hydrophobic amino acids exist in the amino terminus and that the amino-terminal fragment localized in the mitochondria suggests that the cDNA transcript is specific to the mouse mitoribosome. The other newly annotated genes are summarized with their gene names in Table 1. Furthermore, cDNAs of 3 clones shown in Table 1, whose reading frame and start codon were found to be a complete match to the RIKEN full-length cDNA clones, did not show significant similarity to other eukaryotic cells. This indicates that these 3 clones are novel proteins localized in mitochondria.

**Table 1**

| Category of sense cDNAs | Clone No. |
|---|---|
| Identical to Mouse Protein | |
| Malate dehydrogenase | 11, 52, 53, 54 |
| Cytochrome c oxidase, subunit Vb (Cox5b) | 20 |
| ATP synthetase alpha subunit | 23, 27, 84, 85, 95 |
| Uncoupling protein 2 (Ucp2) | 35 |
| Glutaryl-CoA dehydrogenase (Gcdh) | 40, 43, 49, 57, 93 |
| Acetyl-coenzyme A dehydrogenase (Acad1) | 58 |
| Cytochrome b | 1 |
| Aldehyde dehydrogenase 2 | 140 |
| ATP synthetase H+ transporting, mitochondrial F1 complex, | 143 |
| gamma polypeptide 1 | |
| Mitochondrial ribosomal protein S11 | 147 |
| Similar to mouse gene | |
| Phosphoenolpyruvate carboxykinase 2 | 71, 100 |
| 60S ribosomal protein L3 (L4) | 108 |
| NADH-ubiquinone oxidoreductase 13 kDa-A subunit | 144, 150 |
| Inorganic phosphatase | 148 |
| Homologue to mammal gene | |
| Putative cytochrome c oxidase assembly protein | 10, 94 |
| (*Schizosaccharomyces pombe*, 23%) | |
| Heat shock protein 75 (*Homo sapiens*, 89%) | 46, 70, 77 |
| Ribosomal protein S 18 (Rsp 18) (*Homo sapiens,* 76%) | 51, 63 |
| Membrane associated protein SLP-2 | 87 |
| (*Homo sapiens,* 93%) | |
| Mitochondrial 28S ribosomal protein S18-1 | 92 |
| (*Homo sapiens*, 77%) | |
| NADH-ubiquinone oxidoreductase 30 kDa subunit precursor | 99 |
| (*Homo sapiens*, 88%) | |
| Succinate dehydrogenase complex, subunit B, iron | 135 |
| sulfur (*Homo sapiens*, 91 %) | |
| Biphenyl hydrolase-related protein | 145 |
| (*Homo sapiens*, 75%) | |
| Predicted protein | |
| GI: 12852607 | 16 |
| GI: 12840016 | 33,37 |
| GI: 12859851 | 59,72,82 |

### 3. Conclusions

The above results suggest that the analysis method of the present invention enables the provision of a rapid approach for identifying novel gene products that are localized in the mitochondria, and for annotating their functions. The high-throughput screening technology also allows easy identification of groups of proteins localized in organelles such as nucleus, endoplasmic reticulum, Golgi body or peroxisome, by using respective signals. Because of the simplicity of the present method, one skilled in the art capable of constructing a cDNA library and equipped with a FACS facility would be able to perform the technology without resorting to excessive tests. Furthermore, the combination of the present method with a cDNA subtraction method gives more flexibility in that, for example, comparison of expression genes under normal conditions and disease conditions or comparison of expression genes of different tissues, is made possible.

### Industrial Applicability

As described in detail above, the invention of the present application provides a novel method for simple and accurate analysis of the localization of protein, which is applicable to all organelles, and a material for analysis for such method.

## Claims

1. A method for analyzing protein localization which enables one to determine whether or not a test protein localizes to an organelle and comprises the following steps:
(a) a step of introducing a fusion peptide (a), which comprises one half-peptide of an intein, one half-peptide of a fluorescent protein and an organelle-targeting signal peptide, into a eukaryotic cell other than a human embryonic cell;
(b) a step of introducing a test protein bound to a fusion peptide (b), which comprises the other half-peptide of the fluorescent protein and the other half-peptide of the intein, into the eukaryotic cell; and
(c) a step of detecting fluorescence signal emitted by the fluorescent protein.

2. The method of Claim 1, wherein,
in step (a), two or more types of fusion peptide (a), each comprising one half-peptide of different fluorescent proteins and different organelle-targeting signal peptides, are introduced into a eukaryotic cell other than a human embryonic cell;
in step (b), two or more types of fusion peptides (b), each comprising the other half-peptide of the different fluorescent proteins, and each bound to a test protein, is introduced into the eukaryotic cell; and
in step (c), the fluorescent signal is detected.

3. The method of Claim 1 or 2, wherein, in step (a), the fusion peptide (a) is introduced into a eukaryotic cell other than a human embryonic cell by transfecting a recombinant vector (A), which expresses the fusion peptide (a), into the eukaryotic cell and/or;
in step (b), the test protein and the fusion peptide (b) are introduced into a eukaryotic cell other than a human embryonic cell by transfecting a recombinant vector (B), which expresses the fusion peptide (b) and the test protein as a unit, into the eukaryotic cell.

4. A fusion peptide (a), which comprises a half-peptide of an intein, a half-peptide of a fluorescent protein and an organelle targeting signal peptide.

5. A fusion peptide (b), which comprises a half-peptide of a fluorescent protein and a half-peptide of an intein.

6. A recombinant vector (A), which expresses a fusion peptide (a) comprising a half-peptide of an intein, a half-peptide of a fluorescent protein and an organelle targeting signal peptide.

7. A recombinant vector (B), which expresses a fusion peptide (b) comprising a half-peptide of a fluorescent protein and a half-peptide of an intein, and an arbitrary test protein bound thereto.

8. A probe set for analyzing organelle-localized protein, comprising the fusion peptide (a) of Claim 4 or the recombinant vector (A) of Claim 6, and the fusion peptide (b) of Claim 5 or the recombinant vector (B) of Claim 7.

9. The probe set according to Claim 8, wherein
the fusion peptide (a) or the fusion peptide (a) expressed by the recombinant vector (A) comprises two or more types of fusion peptides, each fusion peptide comprising one half-peptide of a fluorescent protein having different signal characteristics and a different organelle targeting signal peptide; and
the fusion peptide (b) or the fusion peptide (b) expressed by the recombinant vector (B) comprises two or more types of fusion peptides, each fusion peptide comprising the other half of the fluorescent protein.

10. A eukaryotic cell other than a human embryonic cell, containing a fusion peptide (a), which comprises a half-peptide of an intein, a half-peptide of a fluorescent protein and an organelle targeting signal peptide.

11. A eukaryotic cell other than a human embryonic cell, comprising two or more types of fusion peptide (a), wherein each fusion peptide comprises one half-peptide of a fluorescent protein and an organelle targeting signal peptide, the fluorescent protein of each fusion peptide have different signal characteristics and the organelle targeting signal peptide of each fusion peptide target different organelle.

12. A kit, comprising two or more of the eukaryotic cells of Claim 10 or 11.

13. The kit of Claim 12 wherein said eukaryotic cells are suspended in liquid medium or immobilized in the form of a cell chip.

14. The kit of claim 12 or 13 wherein said eukaryotic cells are the same kind of cells or a combination of normal cells and diseased cells.

15. A method for analyzing protein localization which enables one to determine whether or not a test protein localizes to an organelle and comprises the following steps:
(a) a step of introducing into a cell as defined in claim 10 or 11 a test protein bound to a fusion peptide (b), which comprises the other half-peptide of the fluorescent protein and the other half-peptide of the intein; and
(b) a step of detecting fluorescence signal emitted by the fluorescent protein.

## Patentansprüche

1. Ein Verfahren zur Untersuchung einer Proteinlokalisierung, welches ermöglicht, zu bestimmen, ob ein Testprotein an eine Organelle lokalisiert oder nicht, und welches die folgenden Schritte umfasst:
(a) ein Schritt, bei dem ein Fusionspeptid (a), das ein Halbpeptid eines Inteins, ein Halbpeptid eines Fluoreszenzproteins und ein Organellen-zielgerichtetes Signalpeptid umfasst, in eine eukaryotische Zelle, die von einer menschlichen embryonalen Zelle verschieden ist, eingeführt wird,
(b) ein Schritt, bei dem ein Testprotein, das an ein Fusionspeptid (b) gebunden ist, das das andere Halbpeptid des Fluoreszenzproteins und das andere Halbpeptid des Inteins umfasst, in die eukaryotische Zelle eingeführt wird, und
(c) ein Schritt, bei dem das vom Fluoreszenzprotein emittierte Fluoreszenzsignal erfasst wird.

2. Das Verfahren gemäß Anspruch 1, wobei
in Schritt (a) zwei oder mehrere Arten Fusionspeptid (a), die jeweils ein Halbpeptid unterschiedlicher Fluoreszenzproteine und unterschiedliche Organellen-zielgerichtete Signal peptide umfassen, in eine eukaryotische Zelle, die von einer menschlichen embryonalen Zelle verschieden ist, eingeführt werden,
in Schritt (b) zwei oder mehrere Arten von Fusionspeptiden (b), die jeweils das andere Halbpeptid der unterschiedlichen Fluoreszenzproteine umfassen und jeweils an ein Testprotein gebunden sind, in die eukaryotische Zelle eingeführt werden, und
in Schritt (c) das Fluoreszenzsignal erfasst wird.

3. Das Verfahren gemäß Anspruch 1 oder 2, wobei
in Schritt (a) das Fusionspeptid (a) in eine eukaryotische Zelle, die von einer menschlichen embryonalen Zelle verschieden ist, eingeführt wird durch Transfektion eines rekombinanten Vektors (A), der das Fusionspeptid (a) exprimiert, in die eukaryotische Zelle, und/oder
in Schritt (b) das Testprotein und das Fusionspeptid (b) in eine eukaryotische Zelle, die von einer menschlichen embryonalen Zelle verschieden ist, eingeführt wird durch Transfektion eines rekombinanten Vektors (B), der das Fusionspeptid (b) und das Testprotein als eine Einheit exprimiert, in die eukaryotische Zelle.

4. Ein Fusionspeptid (a), das ein Halbpeptid eines Inteins, ein Halbpeptid eines Fluoreszenzproteins und ein Organellen-zielgerichtetes Signalpeptid umfasst.

5. Ein Fusionspeptid (b), das ein Halbpeptid eines Fluoreszenzproteins und ein Halbpeptid eines Inteins umfasst.

6. Ein rekombinanter Vektor (A), der ein Fusionspeptid (a) exprimiert, das ein Halbpeptid eines Inteins, ein Halbpeptid eines Fluoreszenzproteins und ein Organellen-zielgerichtetes Signalpeptid umfasst.

7. Ein rekombinanter Vektor (B), der ein Fusionspeptid (b) exprimiert, das ein Halbpeptid eines Fluoreszenzproteins und ein Halbpeptid eines Inteins und ein daran gebundenes willkürliches Testprotein umfasst.

8. Ein Prüfsatz zur Untersuchung von organellenlokalisiertem Protein, umfassend das Fusionspeptid (a) gemäß Anspruch 4 oder den rekombinanten Vektor (A) gemäß Anspruch 6 und das Fusionspeptid (b) gemäß Anspruch 5 oder den rekombinanten Vektor (B) gemäß Anspruch 7.

9. Der Prüfsatz gemäß Anspruch 8, wobei
das Fusionspeptid (a) oder das Fusionspeptid (a), das durch den rekombinanten Vektor (A) exprimiert wird, zwei oder mehrere Arten von Fusionspeptiden umfasst, wobei jedes Fusionspeptid ein Halbpeptid eines Fluoreszenzproteins mit unterschiedlichen Signaleigenschaften und ein unterschiedliches Organellen-zielgerichtetes Signalpeptid umfasst, und das Fusionspeptid (b) oder das Fusionspeptid (b), das durch den rekombinanten Vektor (B) exprimiert wird, zwei oder mehrere Arten von Fusionspeptiden umfasst, wobei jedes Fusionspeptid die andere Hälfte des Fluoreszenzproteins umfasst.

10. Eine eukaryotische Zelle, die von einer menschlichen embryonalen Zelle verschieden ist, umfassend ein Fusionspeptid (a), das ein Halbpeptid eines Inteins, ein Halbpeptid eines Fluoreszenzproteins und ein Organellen-zielgerichtetes Signalpeptid umfasst.

11. Eine eukaryotische Zelle, die von einer menschlichen embryonalen Zelle verschieden ist, umfassend zwei oder mehrere Arten Fusionspeptid (a), wobei jedes Fusionspeptid ein Halbpeptid eines Fluoreszenzproteins und ein Organellen-zielgerichtetes Signalpeptid umfasst, die jeweiligen Fluoreszenzproteine der Fusionspeptide unterschiedliche Signaleigenschaften haben und die jeweiligen Organellen-zielgerichteten Signalpeptide der Fusionspeptide auf unterschiedliche Organellen zielgerichtet sind.

12. Ein Kit, umfassend zwei oder mehrere der eukaryotischen Zellen gemäß Anspruch 10 oder 11.

13. Das Kit gemäß Anspruch 12, wobei die eukaryotischen Zellen in einem flüssigen Medium suspendiert oder in Form eines Zellchips immobilisiert sind.

14. Das Kit gemäß Anspruch 12 oder 13, wobei die eukaryotischen Zellen die gleiche Art Zellen sind oder eine Kombination aus normalen Zellen und erkrankten Zellen sind.

15. Ein Verfahren zur Untersuchung einer Proteinlokalisierung, welches ermöglicht, zu bestimmen, ob ein Testprotein an eine Organelle lokalisiert oder nicht, und welches die folgenden Schritte umfasst:
(a) ein Schritt, bei dem ein Testprotein, das an ein Fusionspeptid (b) gebunden ist, welches das andere Halbpeptid des Fluoreszenzproteins und das andere Halbpeptid des Inteins umfasst, in eine wie in Anspruch 10 oder 11 definierte Zelle eingeführt wird, und
(b) ein Schritt, bei dem das vom Fluoreszenzprotein emittierte Fluoreszenzsignal erfasst wird.

## Revendications

1. Procédé pour analyser la localisation d'une protéine qui permet de déterminer si une protéine d'essai est localisée dans un organite et comprend les étapes suivantes :
(a) une étape d'introduction d'un peptide de fusion (a), qui comprend un demi peptide d'une intéine, un demi peptide d'une protéine fluorescente et un peptide signal ciblant un organite, dans une cellule eucaryote autre qu'une cellule embryonnaire humaine ;
(b) une étape d'introduction d'une protéine d'essai liée à un peptide de fusion (b), qui comprend l'autre demi peptide de la protéine fluorescente et l'autre demi peptide de l'intéine, dans la cellule eucaryote ; et
(c) une étape de détection du signal de fluorescence émis par la protéine fluorescente.

2. Procédé selon la revendication 1, dans lequel,
dans l'étape (a) deux ou plusieurs types de peptide de fusion (a), chacun comprenant un demi peptide de différentes protéines fluorescente et différents peptides signal ciblant un organite, sont introduits dans une cellule eucaryote autre qu'une cellule embryonnaire humaine ;
dans l'étape (b), deux ou plusieurs types de peptide de fusion (b), comprenant chacun l'autre demi peptide de différentes protéines de fusion fluorescente, et chacun étant lié à une protéine d'essai, sont introduits dans la cellule eucaryote ; et
dans l'étape (c), le signal fluorescent est détecté.

3. Procédé selon la revendication 1 ou 2, dans lequel, dans l'étape (a), le peptide de fusion (a) est introduit dans une cellule eucaryote autre qu'une cellule embryonnaire humaine par transfection d'un vecteur recombinant (A), qui exprime le peptide de fusion (a) ; dans la cellule eucaryote et/ou ;
dans l'étape (b), la protéine d'essai et le peptide de fusion (b) sont introduits dans une cellule eucaryote autre qu'une cellule embryonnaire humaine par transfection d'un vecteur recombinant (B), qui exprime le peptide de fusion (b) et la protéine d'essai sous forme d'une unité, dans la cellule eucaryote.

4. Peptide de fusion (a), qui comprend un demi peptide d'une intéine, un demi peptide d'une protéine fluorescente et un peptide signal ciblant un organite.

5. Peptide de fusion (b), qui comprend un demi peptide d'une protéine fluorescente et un demi peptide d'une intéine.

6. Vecteur recombinant (A), qui exprime un peptide de fusion (a) comprenant un demi peptide d'une intéine, un demi peptide d'une protéine fluorescente et un peptide signal ciblant un organite.

7. Vecteur recombinant (B), qui exprime un peptide de fusion (b) comprenant un demi peptide d'une protéine fluorescente et un demi peptide d'une intéine, et une protéine d'essai arbitraire leur étant liée.

8. Ensemble de sonde pour analyser une protéine localisée dans un organite, comprenant le peptide de fusion (a) selon la revendication 4 ou le vecteur recombinant (A) selon la revendication 6, et le peptide de fusion (b) selon la revendication 5 ou le vecteur recombinant (B) selon la revendication 7.

9. Ensemble de sonde selon la revendication 8, dans lequel
le peptide de fusion (a) ou le peptide de fusion (a) exprimé par le vecteur recombinant (A) comprend deux ou plusieurs types de peptide de fusion, chaque peptide de fusion comprenant un demi peptide d'une protéine fluorescente ayant différentes caractéristiques de signal et un peptide signal ciblant un organite différent ; et
le peptide de fusion (b) ou le peptide de fusion (b) exprimé par le vecteur recombinant (B) comprend deux ou plusieurs types de peptide de fusion, chaque peptide de fusion comprenant l'autre moitié de la protéine fluorescente.

10. Cellule eucaryote autre qu'une cellule embryonnaire humaine, contenant un peptide de fusion (a), qui comprend un demi peptide d'intéine, un demi peptide d'une protéine fluorescente et un peptide signal ciblant un organite.

11. Cellule eucaryote autre qu'une cellule embryonnaire humaine, comprenant deux ou plusieurs types de peptide de fusion (a), où chaque peptide de fusion comprend un demi peptide d'une protéine fluorescente et un peptide signal ciblant un organite, la protéine fluorescente de chaque peptide de fusion ayant différentes caractéristiques de signal et le peptide signal ciblant un organite de chaque peptide de fusion ciblant différents organites.

12. Ensemble, comprenant deux ou plusieurs des cellules eucaryotes selon la revendication 10 ou 11.

13. Ensemble selon la revendication 12, dans lequel lesdites cellules eucaryote sont mises en suspension dans un milieu liquide ou sont immobilisées sous la forme d'une puce cellulaire.

14. Ensemble selon la revendication 12 ou 13, dans lequel lesdites cellules eucaryotes sont de type identique de cellules ou une combinaison de cellules normales et de cellules malades.

15. Procédé pour analyser la localisation d'une protéine qui permet de déterminer si une protéine d'essai est localisée dans un organite et comprend les étapes suivantes :
(a) une étape d'introduction dans une cellule selon la revendication 10 ou 11, une protéine d'essai liée à un peptide de fusion (b), qui comprend l'autre demi peptide de la protéine de fusion et l'autre demi peptide de l'intéine ; et
(b) une étape de détection du signal de fluorescence émis par la protéine fluorescente.
